# FASCICULE DE BREVET EUROPEEN

(11) **EP 2 070 514 B1**
(45) Date de publication et mention de la délivrance du brevet: **12.04.2017**
(21) Numéro de dépôt: 08305805.7
(22) Date de dépôt: 14.11.2008
(51) Int. Cl.: A61K 8/68, A61Q 5/04, A61Q 5/12

(54) **Procédé capillaire de traitement durable de la fibre en quatre étapes**
Verfahren zur dauerhaften Haarbehandlung von Fasern in vier Schritte
Method for long-lasting hair treatment of fibre in four steps

(30) Priorité: 15.11.2007 FR 0759087
(43) Date de publication de la demande: 17.06.2009
(73) Titulaire: L'Oréal, 75008 Paris (FR)
(72) Inventeur: Pataut, Françoise, Tokyo Kanagawa 102-0082 (JP); Itaya, Ayako, Kawasaki City Kanagawa 216-0035 (JP)
(74) Mandataire: Caillet, Isabelle

(56) Documents cités:
- EP-A- 0 278 505
- EP-A- 0 680 743
- EP-A- 1 181 925
- EP-A- 1 277 459
- US-A1- 2005 013 786
- US-B1- 6 312 674

## Description

La présente invention se rapporte à un procédé de traitement capillaire comprenant une étape d'application d'une composition alcaline faiblement réductrice et une étape d'application d'une composition ayant un pH acide contenant un céramide, et son utilisation pour assouplir les cheveux de façon durable.

Certains cheveux, en particulier les cheveux asiatiques, sont naturellement durs et raides.

Les femmes souhaiteraient casser cette raideur sans pour autant donner une forme différente, c'est-à-dire bouclée, à la chevelure.

On connaît des procédés de traitement permettant d'assouplir les cheveux. Ces procédés utilisent des agents conditionnant ou consistent en des traitements sans rinçage contenant des silicones ou des polymères cationiques.

Ces procédés peuvent conduire à un effet d'assouplissement de la chevelure, mais cet effet est non durable.

Il existe donc un besoin de disposer d'un procédé de traitement capillaire qui permette d'assouplir la fibre capillaire de façon durable, sans pour autant donner une forme différente à la chevelure. Ce procédé ne doit pas affecter le toucher de la chevelure, qui doit rester naturel, c'est-à-dire ni gras ni chargé.

La demanderesse a découvert que l'utilisation d'un procédé de traitement capillaire comprenant une étape d'application d'une composition alcaline faiblement réductrice et une étape d'application d'une composition ayant un pH acide contenant un céramide, permettait de résoudre les problèmes de l'art antérieur et d'assouplir la fibre capillaire de façon durable.

On connaît du document EP 1468667 A1 un procédé de traitement capillaire pour le lissage des cheveux comprenant une étape d'application sur les cheveux d'une composition contenant un céramide, et une étape d'élévation de la température des cheveux.

On connaît également du document FR 2767486 A1 un procédé de traitement pour la déformation permanente des matières kératiniques comprenant l'application sur les matières kératiniques d'une composition contenant de la cystéïne et/ou de la cystéamine.

Enfin, on connaît du document JP2005-162699 un procédé de traitement pour les cheveux qui peut comporter une étape de réduction et une étape d'oxydation. La composition oxydante comprend nécessairement un oxydant tel que l'eau oxygénée ou les bromates. Cette composition oxydante peut comprendre en outre des agents modificateurs de pH. Le document JP2005-162699 cite les céramides comme de simples adjuvants pouvant être utilisés aussi bien dans la composition oxydante que dans la composition réductrice. Toutefois, aucun exemple ne divulgue l'utilisation de céramides. Ce document ne divulgue pas un procédé de traitement comprenant une étape de réduction suivie d'une étape d'application d'une composition ayant un pH acide comprenant un céramide.

Ces procédés ne donnent pas satisfaction pour assouplir la fibre capillaire de façon durable.

L'invention a donc pour objet un procédé de traitement capillaire comprenant les étapes consistant à :
- appliquer sur les cheveux une composition alcaline comprenant dans un milieu cosmétiquement acceptable un ou plusieurs agents réducteurs représentant entre 0,1 et 4% en poids du poids total de la composition alcaline,
- rincer les cheveux,
- appliquer sur les cheveux une composition ayant un pH acide contenant dans un milieu cosmétiquement acceptable un ou plusieurs céramides, ladite composition étant exempte de tout agent oxydant, puis
- rincer les cheveux.

Le ou les agents réducteurs présents dans la composition alcaline utilisée selon l'invention représentent entre 0,1 et 4%, mieux entre 1 et 2%, en poids du poids total de la composition alcaline.

Le ou les agents réducteurs peuvent être choisis parmi tous les agents réducteurs habituellement utilisés dans les compositions cosmétiques.

Ainsi, le ou les agents réducteurs utilisables dans la présente invention sont généralement choisis parmi les agents réducteurs de formule :

H(X')_{q}(R⁰)ᵣ

dans laquelle X' représente P, S ou SO₂, q vaut 0 ou 1, r vaut 1 ou 2 et R⁰ est un radical (C₁-C₂₀)hydrocarboné, linéaire, ramifié, saturé, insaturé, éventuellement interrompu par un hétéroatome, et comportant éventuellement des substituants choisis parmi un groupement hydroxy, un groupement halogéné, un groupement amine ou un groupement carboxy, un groupement ((C₁-C₃₀) alcoxy)carbonyle, un groupement amido, un groupement ((C₁₋C₃₀)alkyl)amino carbonyle, un groupement (C₁-C₃₀₎acyle) amino, un groupement mono ou dialkylamino, un groupement mono ou dihydroxylamino, ou l'un de ses sels en combinaison avec une base.

On peut citer à titre d'agents réducteurs l'acide thioglycolique, l'acide thiolactique, le monothioglycolate de glycérol, la cystéamine, la N-acétyl-cystéamine, la N-propionyl-cystéamine, la cystéine, la N-acétyl-cystéine, l'acide thiomalique, la panthétéine, l'acide 2,3-dimercaptosuccinique, les N-(mercaptoalkyl)-ω-hydroxyalkylamides, les N-mono ou N,N-dialkylmercapto-4-butyramides, les aminomercapto-alkylamides, les dérivés des acides N-(mercaptoalkyl)succinamiques et des N-(mercaptoalkyl)succinimides, les alkylamino mercaptoalkylamides, le mélange azéotrope de thioglyconate de 2-hydroxypropyle et de thioglycolate de (2-hydroxy-1-méthyl)éthyle, les mercaptoalkylaminoamides, les N-mercapto-alkylalcanediamides et les dérivés d'acide formamidine sulfinique, et leurs sels.

Selon un mode de réalisation préféré de l'invention, le ou les agents réducteurs sont choisis parmi la cystéine ou la N-acétyl-cystéine.

Outre le ou les agents réducteurs, la composition alcaline utilisée selon l'invention peut comprendre un ou plusieurs actifs cosmétiques choisis parmi les agents gélifiants et/ou épaississants, les glycols, les agents de gonflement et de pénétration permettant de renforcer l'efficacité du réducteur, les silicones, et les parfums.

Le ou les agents gélifiants et/ou épaississants peuvent être choisis parmi les agents épaississants cellulosiques, par exemple l'hydroxyéthylcellulose, l'hydroxypropylcellulose, le carboxyméthylcellulose et les hydroxypropyl méthylcellulose, tel que l'AQU D-3295A commercialisé par HERCULES, la gomme de guar et ses dérivés, par exemple l'hydroxypropyl guar, commercialisé par la société RHODIA sous la référence JAGUAR HP 105, les gommes d'origine microbienne, telle que la gomme de xanthane et la gomme de scléroglucane, la gomme de caroube, les dérivés de chitine et de chitosane, les carraghénanes, les argiles, les poly(oxyalkylène)glycols, les esters de poly(oxyalkylène)glycols, les homopolymères réticulés d'acide acrylique ou d'acide acrylamidopropanesulfonique, par exemple le CARBOMER, les copolymères acrylates/C₁-C₃₀ alkyl acrylates, par exemple ceux de type PEMULEN, les copolymères d'acrylate d'ammonium/acrylamide, tel que le BOZEPOL C commercialisé par HOECHST, les copolymères acrylamide/acrylamido-2-méthyl propane sulfonique, tel que le SEPIGEL 305 commercialisé par SEPPIC, les copolymères acrylate de sodium/acrylamide, tel que le SEPIGEL 901 commercialisé par SEPPIC, les copolymères chlorure de méthacrylate de triméthyl éthyl ammonium/ acrylate, tel que le SALCARE SC 92 commercialisé par ALLIED COLLOIDS, et les homopolymères de chlorure de méthacrylate d'éthyle triméthyl ammonium, tel que le SALCARE SC 95 commercialisé par ALLIED COLLOIDS.

Les silicones éventuellement présentes dans la composition alcaline utilisée dans le procédé selon l'invention sont en particulier des polyorganosiloxanes insolubles dans la composition et peuvent se présenter sous forme d'huiles, de cires, de résines ou de gommes.

Les organopolysiloxanes sont définis plus en détail dans l'ouvrage de Walter NOLL "Chemistry and Technology of Silicones" (1968) Academie Press.

Les silicones peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :
(i) les silicones cycliques comportant de 3 à 7 atomes de silicium et de préférence 4 à 5.
   Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHONE POULENC, le décaméthylcyclopentasiloxane commercialisé sous le nom de "VOLATILE SILICONE 7158" par UNION CARBIDE, "SILBIONE 70045 V 5" par RHONE POULENC, ainsi que leurs mélanges.
   On peut également citer les cyclocopolymères du type diméthylsiloxanes/ méthylalkylsiloxane, tel que la "SILICONE VOLATILE FZ 3109" commercialisée par la société UNION CARBIDE, de structure chimique : 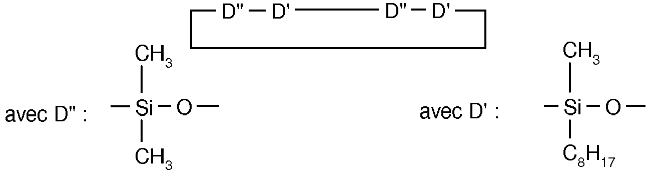
   On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25°C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, P. 27-32 - TODD & BYERS "Volatile Silicone fluids for cosmetics".

On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyle ayant une viscosité de 5.10⁻⁶ à 2,5 m²/s à 25 °C et de préférence 1.10⁻⁵ à 1 m²/s. La viscosité des silicones est par exemple mesurée à 25 °C selon la norme ASTM 445 Appendice C.

Parmi ces polyalkylsiloxanes, on peut citer à titre non limitatif les produits commerciaux suivants :
- les huiles SILBIONE des séries 47 et 70 047 ou les huiles MIRASIL commercialisées par RHONE POULENC telles que par exemple l'huile 70 047 V 500 000 ;
- les huiles de la série MIRASIL commercialisées par la société RHONE POULENC ;
- les huiles de la série 200 de la société DOW CORNING telles que plus particulièrement la DC200 de viscosité 60 000 Cst ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHONE POULENC.

On peut également citer les polydiméthylsiloxanes à groupements aminoéthyl aminopropyl et alpha-oméga silanols.

Dans cette classe de polyalkylsiloxanes, on peut également citer les produits commercialisés sous les dénominations "ABIL WAX 9800 et 9801" par la société GOLDSCHMIDT qui sont des polyalkyl (C₁-C₂₀) siloxanes.

Les polyalkylarylsiloxanes sont particulièrement choisis parmi les polydiméthyl méthylphénylsiloxanes, les polydiméthyl diphénylsiloxanes linéaires et/ou ramifiés de viscosité de 1.10⁻⁵ à 5.10⁻²m²/s à 25 °C.

Parmi ces polyalkylarylsiloxanes on peut citer à titre d'exemple les produits commercialisés sous les dénominations suivantes :
- les huiles SILBIONE de la série 70 641 de RHONE POULENC ;
- les huiles des séries RHODORSIL 70 633 et 763 de RHONE POULENC ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Les gommes de silicone pouvant être présentes dans la composition alcaline du procédé selon l'invention sont notamment des polydiorganosiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- les gommes polydiméthylsiloxane,
- les gommes polydiméthylsiloxanes/méthylvinylsiloxane,
- les gommes polydiméthylsiloxane/diphénylsiloxane,
- les gommes polydiméthylsiloxane/phénylméthylsiloxane,
- les gommes polydiméthylsiloxane/diphénylsiloxane/méthylvinylsiloxane. Des produits plus particulièrement utilisables sont les mélanges suivants:
   - les mélanges formés à partir d'un polydiméthylsiloxane hydroxylé en bout de chaîne (dénommé diméthiconol selon la nomenclature du dictionnaire CTFA) et d'un poly-diméthylsiloxane cyclique (dénommé cyclométhicone selon la nomenclature du dictionnaire CTFA) tel que le produit Q2 1401 commercialisé par la société DOW CORNING ;
   - les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
   - les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes éventuellement présentes dans la composition alcaline du procédé selon l'invention sont des systèmes siloxaniques réticulés renfermant les unités : R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées éventuellement présentes dans la composition alcaline du procédé selon l'invention sont des silicones telles que définies précédemment et comportant dans leur structure un ou plusieurs groupements organofonctionnels fixés par l'intermédiaire d'un radical hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET L 722, L 7500, L 77, L 711 de la société UNION CARBIDE et l'alkyl (C₁₂) méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. On peut encore citer les quaternium-80, commercialisé par la société GOLDSCHMIDT sous les références ABIL QUAT 3270, ABIL QUAT 3272 et ABIL QUAT 3274. Les groupements aminés substitués sont en particulier des groupements aminoalkyle en C₁-C₄ ; on peut citer les silicones aminées comportant des groupements alcoxy telle que la silicone BELSIL ADM LOG 1 commercialisé par la société WACKER ;
- des groupements thiols comme les produits commercialisés sous les dénominations "GP 72 A" et "GP 71" de GENESEE ;
- des groupements alcoxylés comme le produit commercialisé sous la dénomination "SILICONE COPOLYMER F-755" par SWS SILICONES et ABIL WAX 2428, 2434 et 2440 par la société GOLDSCHMIDT ;
- des groupements hydroxylés comme les polyorganosiloxanes à fonction hydroxyalkyle décrits dans la demande de brevet français FR-A-85 16334 ;
- des groupements acyloxyalkyle tels que par exemple les polyorganosiloxanes décrits dans le brevet US-A-4957732 ;
- des groupements anioniques du type carboxylique comme par exemple dans les produits décrits dans le brevet EP 186 507 de la société CHISSO CORPORATION, ou de type alkylcarboxyliques comme ceux présents dans le produit X-22-3701 E de la société SHIN-ETSU ; 2-hydroxyalkylsulfonate ; 2-hydroxyalkylthiosulfate tels que les produits commercialisés par la société GOLDSCHMIDT sous les dénominations "ABIL S201" et "ABIL S255" ;
- des groupements hydroxyacylamino, comme les polyorganosiloxanes décrits dans la demande EP 342 834. On peut citer par exemple le produit Q2-8413 de la société DOW CORNING.

A titre de silicone non ionique, on peut citer le polymère block poly diméthylsiloxane/aminopolyéther (80/20).

Le pH de la composition alcaline est généralement compris entre 7,5 et 9,5.

Le pH de la composition alcaline est ajusté au moyen d'un ou plusieurs agents alcalins, tels que par exemple l'ammoniaque, la monoéthanolamine, la diéthanolamine, la triéthanolamine, l'isopropanolamine, la propanediamine-1,3, la lysine, l'arginine, un carbonate ou bicarbonate alcalin ou d'ammonium, un carbonate organique tel que le carbonate de guanidine, ou bien encore un hydroxyde alcalin.

Comme expliqué précédemment, le procédé selon l'invention comprend encore une étape d'application sur les cheveux d'une composition ayant un pH acide contenant dans un milieu cosmétiquement acceptable un ou plusieurs céramides.

Le ou les céramides présents dans la composition ayant un pH acide utilisée selon l'invention peuvent être choisis parmi les composés de formule (I) suivante : 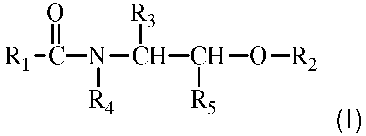 dans laquelle :
- R₁ désigne soit un radical hydrocarboné, linéaire ou ramifié, saturé ou insaturé en C₉-C₃₀, ce radical pouvant être substitué par un ou plusieurs groupements hydroxyle, ces groupements hydroxyle étant éventuellement estérifiés par un acide gras saturé ou insaturé en C₁₆-C₃₀ ; soit un radical R"-(NR-CO)-R', où R désigne un atome d'hydrogène ou un radical hydrocarboné en C₁-C₁₀ mono ou polyhydroxylé, préférentiellement monohydroxylé, R' et R" sont des radicaux hydrocarbonés dont la somme des atomes de carbone est comprise entre 9 et 30, R' étant un radical divalent ;
- R₂ désigne un atome d'hydrogène ou un radical (glycosyle)ₙ, (galactosyle)ₘ ou sulfogalactosyle, dans lesquels n est un nombre entier variant de 1 à 4 inclusivement et m est un nombre entier variant de 1 à 8 inclusivement ;
- R₃ désigne un atome d'hydrogène ou un radical hydrocarboné en C₁₆-C₂₇, saturé ou insaturé, ce radical pouvant être substitué par un ou plusieurs radicaux alkyle en C₁-C₁₄ ; R₃ peut également désigner un radical α-hydroxyalkyle en C₁₅-C₂₆ dont le groupement hydroxyle peut éventuellement être estérifié par un α-hydroxyacide en C₁₆-C₃₀ ;
- R₄ désigne un atome d'hydrogène, un radical hydrocarboné en C₁₆-C₂₇, saturé ou insaturé, ou un radical -CH₂-CHOH-CH₂-O-R₆ dans lequel R₆ désigne un radical hydrocarboné en C₁₀-C₂₆ ;
- R₅ désigne un atome d'hydrogène ou un radical hydrocarboné en C₁-C₄ mono ou polyhydroxylé.

De préférence, le ou les céramides sont choisis parmi les composés de formule (I) dans laquelle R₁ désigne un radical alkyle saturé ou insaturé dérivé d'acides gras en C₁₆-C₂₂ éventuellement hydroxylé ; R₂ désigne un atome d'hydrogène ; et R₃ désigne un radical linéaire saturé en C₁₅ éventuellement hydroxylé.

On peut citer en particulier :
- la N-linoléoyldihydrosphingosine,
- la N-oléoyldihydrosphingosine,
- la N-palmitoyldihydrosphingosine,
- la N-stéaroyldihydrosphingosine,
- la N-béhénoyldihydrosphingosine,
- la N-2-hydroxypalmitoyldihydrosphingosine,
- la N-stéaroylphytosphingosine,
ou les mélanges de ces composés.

Selon un mode de réalisation préféré de l'invention, le céramide est la N-oléoyldihydrosphingosine.

Le ou les céramides représentent généralement de 0,001 à 20%, de préférence de 0,01 à 10%, et mieux de 0,05 à 1% en poids du poids total de la composition.

Outre le ou les céramides, la composition ayant un pH acide utilisée dans le procédé selon l'invention peut comprendre un ou plusieurs actifs cosmétiques choisis parmi les tensioactifs cationiques, anioniques, non-ioniques ou amphotères, les cires, les silicones, les conservateurs et les parfums.

Selon l'invention, les agents tensioactifs peuvent être des agents tensioactifs non ioniques, cationiques, anioniques ou amphotères.

A titre d'exemples d'agents tensioactifs anioniques, on peut citer notamment les sels, en particulier les sels de métaux alcalins tels que les sels de sodium, les sels d'ammonium, les sels d'amines, les sels d'aminoalcools ou les sels de métaux alcalino-terreux, par exemple, de magnésium, des composés suivants : les alkyl-sulfates, les alkyléther-sulfates, les alkylamidoéthersulfates, les alkylarylpolyéthersulfates, les monoglycéride-sulfates, les alkylsulfonates, les phosphates d'alkyle, les alkylamidesulfonates, les alkylarylsulfonates, les α-oléfine-sulfonates, les paraffine-sulfonates, les sulfosuccinates d'alkyle, les alkyléthersulfosuccinates, les alkylamidesulfosuccinates, les sulfoacétates d'alkyle, les acylsarcosinates, et les acylglutamates, les groupes alkyle ou acyle de tous ces composés comportant de 6 à 24 atomes de carbone et le groupe aryle désignant de préférence un groupe phényle ou benzyle.

On peut également utiliser les esters d'alkyle en C₆-C₂₄ et d'acides polyglycoside-carboxyliques tels que les glucoside-citrates d'alkyle, les polyglycoside-tartrates d'alkyle, et les polyglycoside-sulfosuccinates d'alkyle ; les sulfosuccinamates d'alkyle, les iséthionates d'acyle et les N-acyltaurates, le groupe alkyle ou acyle de tous ces composés comportant de 12 à 20 atomes de carbone. Parmi les tensioactifs anioniques encore utilisables, on peut également citer les lactylates d'acyle dont le groupe acyle comporte de 8 à 20 atomes de carbone.

En outre, on peut encore citer les acides d'alkyl-D-galactoside uroniques et leurs sels ainsi que les acides alkyl(C₆-C₂₄)éther-carboxyliques polyoxyalkylénés, les acides alkyl(C₆-C₂₄)aryl(C₆-C₂₄)éther-carboxyliques polyoxyalkylénés, les acides alkyl(C₆-C₂₄)amidoéther carboxyliques polyoxyalkylénés et leurs sels, en particulier ceux comportant de 2 à 50 groupements oxyde d'éthylène, et leurs mélanges.

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178). Ainsi, ils peuvent être notamment choisis parmi les alcools, les alpha-diols, les alkyl(C₁-C₂₀)phénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras, les amides gras polyéthoxylés ayant de préférence de 2 à 30 moles d'oxyde d'éthylène, les amides gras polyglycérolés comportant en moyenne de 1 à 5 et en particulier de 1,5 à 4 groupements glycérol, les esters d'acides gras du sorbitane éthoxylés ayant de 2 à 30 moles d'oxyde d'éthylène, les esters d'acides gras du saccharose, les esters d'acides gras du polyéthylèneglycol, les alkyl(C₆-C₂₄)polyglycosides, les dérivés de N-alkyl(C₆-C₂₄)glucamine, les oxydes d'amines tels que les oxydes d'alkyl(C₁₀-C₁₄)amines ou les oxydes de N-acyl(C₁₀-C₁₄)aminopropylmorpholine, ainsi que des mélanges de ceux-ci.

Les agents tensioactifs amphotères peuvent être notamment des dérivés d'amines aliphatiques secondaires ou tertiaires dans lesquels le groupe aliphatique est une chaîne linéaire ou ramifiée comportant de 8 à 22 atomes de carbone et contenant au moins un groupe anionique hydrosolubilisant tel que, par exemple, un groupe carboxylate, sulfonate, sulfate, phosphate ou phosphonate ; on peut citer encore les alkyl(C₈-C₂₀)bétaïnes, les sulfobétaïnes, les alkyl(C₈-C₂₀)amidoalkyl(C₆-C₈)-bétaïnes ou les alkyl(C₈-C₂₀)amidoalkyl(C₆-C₈)sulfobétaïnes, et leurs mélanges.

Parmi les dérivés d'amines, on peut citer les produits commercialisés sous la dénomination MIRANOL®, tels que ceux décrits dans les brevets US 2,528,378 et US 2,781,354 et classés dans le dictionnaire CTFA, 3ème édition, 1982, sous les dénominations Amphocarboxyglycinate et Amphocarboxypropionate correspondant respectivement aux formules (a) et (b) :

R₂-CONHCH₂CH₂-N⁺(R₃)(R₄)(CH₂COO⁻) (a)

dans laquelle :
R₂ représente un groupe alkyle dérivé d'un acide R₂-COOH présent dans l'huile de coprah hydrolysée, un groupe heptyle, nonyle ou undécyle,
R₃ représente un groupe bêta-hydroxyéthyle, et
R₄ représente un groupe carboxyméthyle ;
et

R₂'-CONHCH₂CH₂-N(B)(C) (b)

dans laquelle :
B représente -CH₂CH₂OX',
C représente -(CH₂)_{z}-Y', avec z = 1 ou 2,
X' représente le groupe -CH₂CH₂-COOH ou un atome d'hydrogène,
Y' représente -COOH ou le groupe -CH₂-CHOH-SO₃H,
R₂' représente un groupe alkyle d'un acide R₂'-COOH présent dans l'huile de coprah ou dans l'huile de lin hydrolysée, un groupe alkyle, notamment en C₁₇ et sa forme iso, ou un groupe en C₁₇ insaturé.

Ces composés sont classés dans le dictionnaire CTFA, 5ème édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé sous la dénomination commerciale MIRANOL® C2M concentré par la société RHODIA.

La composition selon l'invention peut comprendre un ou plusieurs tensioactifs cationiques bien connus en soi, tels que les sels d'amines grasses primaires, secondaires ou tertiaires, éventuellement polyoxyalkylénées, les sels d'ammonium quaternaire, et leurs mélanges.

A titre de sels d'ammonium quaternaires, on peut notamment citer, par exemple :
- ceux qui présentent la formule générale (VI) suivante : 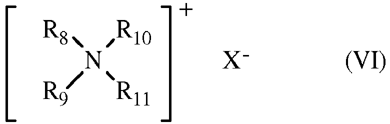 dans laquelle les radicaux R₈ à R₁₁, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre et les halogènes. Les radicaux aliphatiques sont par exemple choisis parmi les radicaux alkyle, alcoxy, polyoxyalkylène (C₂-C₆), alkylamide, alkyl(C₁₂-C₂₂)amidoalkyle(C₂-C₆), alkyl(C₁₂-C₂₂)acétate, hydroxyalkyle, comportant environ de 1 à 30 atomes de carbone ; X est un anion choisi dans le groupe des halogénures, phosphates, acétates, lactates, alkyl(C₂-C₆)sulfates, alkyl- ou alkylaryl-sulfonates ;
- les sels d'ammonium quaternaire de l'imidazoline, comme par exemple ceux de formule (VII) suivante : 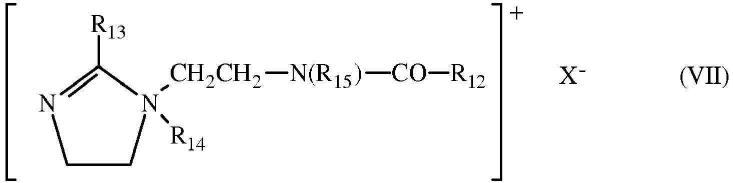 dans laquelle R₁₂ représente un radical alcényle ou alkyle comportant de 8 à 30 atomes de carbone, par exemple dérivés des acides gras du suif, R₁₃ représente un atome d'hydrogène, un radical alkyle en C₁-C₄ ou un radical alcényle ou alkyle comportant de 8 à 30 atomes de carbone, R₁₄ représente un radical alkyle en C₁-C₄, R₁₅ représente un atome d'hydrogène, un radical alkyle en C₁-C₄, X⁻ est un anion choisi dans le groupe des halogénures, phosphates, acétates, lactates, alkylsulfates, alkyl- ou alkylaryl-sulfonates. De préférence, R₁₂ et R₁₃ désignent un mélange de radicaux alcényle ou alkyle comportant de 12 à 21 atomes de carbone, par exemple dérivés des acides gras du suif, R₁₄ désigne un radical méthyle, R₁₅ désigne un atome d'hydrogène. Un tel produit est par exemple commercialisé sous la dénomination REWOQUAT^{®} W 75 par la société REWO ;
- les sels de diammonium quaternaire de formule (VIII) : 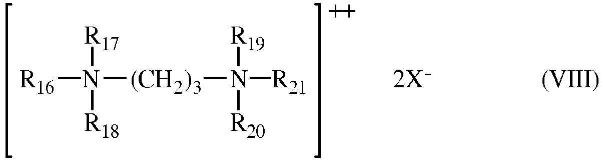 dans laquelle R₁₆ désigne un radical aliphatique comportant environ de 16 à 30 atomes de carbone, R₁₇, R₁₈, R₁₉, R₂₀ et R₂₁, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium ;
- les sels d'ammonium quaternaire contenant au moins une fonction ester, tels que ceux de formule (IX) suivante : 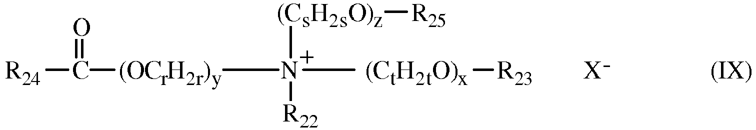 dans laquelle :
   R₂₂ est choisi parmi les radicaux alkyles en C₁-C₆ et les radicaux hydroxyalkyles ou dihydroxyalkyles en C₁-C₆ ;
   R₂₃ est choisi parmi :
      - le radical 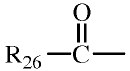
      - les radicaux R₂₇ hydrocarbonés en C₁-C₂₂, linéaires ou ramifiés, saturés ou insaturés,
      - l'atome d'hydrogène,
   R₂₅ est choisi parmi :
      - le radical 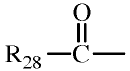
      - les radicaux R₂₉ hydrocarbonés en C₁-C₆, linéaires ou ramifiés, saturés ou insaturés,
      - l'atome d'hydrogène,
   R₂₄, R₂₆ et R₂₈, identiques ou différents, sont choisis parmi les radicaux hydrocarbonés en C₇-C₂₁, linéaires ou ramifiés, saturés ou insaturés;
   r, s et t, identiques ou différents, sont des entiers valant de 2 à 6 ;
   y est un entier valant de 1 à 10 ;
   x et z, identiques ou différents, sont des entiers valant de 0 à 10 ;
   X- est un anion simple ou complexe, organique ou inorganique ;
   sous réserve que la somme x + y + z vaut de 1 à 15, que lorsque x vaut 0 alors R₂₃ désigne R₂₇ et que lorsque z vaut 0 alors R₂₅ désigne R₂₉.

Les radicaux alkyles R₂₂ peuvent être linéaires ou ramifiés et plus particulièrement linéaires.

De préférence R₂₂ désigne un radical méthyle, éthyle, hydroxyéthyle ou dihydroxypropyle, et plus particulièrement un radical méthyle ou éthyle. Avantageusement, la somme x + y + z vaut de 1 à 10.

Lorsque R₂₃ est un radical R₂₇ hydrocarboné, il peut être long et avoir de 12 à 22 atomes de carbone, ou court et avoir de 1 à 3 atomes de carbone.

Lorsque R₂₅ est un radical R₂₉ hydrocarboné, il a de préférence 1 à 3 atomes de carbone.

Avantageusement, R₂₄, R₂₆ et R₂₈, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.

De préférence, x et z, identiques ou différents, valent 0 ou 1. Avantageusement, y est égal à 1.

De préférence, r, s et t, identiques ou différents, valent 2 ou 3, et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X- est encore plus particulièrement le chlorure ou le méthylsulfate.

On utilise plus particulièrement dans la composition selon l'invention, les sels d'ammonium de formule (IX) dans laquelle :
- R₂₂ désigne un radical méthyle ou éthyle,
- x et y sont égaux à 1 ;
- z est égal à 0 ou 1 ;
- r, s et t sont égaux à 2 ;
- R₂₃ est choisi parmi :
   - le radical 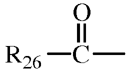
   - les radicaux méthyle, éthyle ou hydrocarbonés en C₁₄-C₂₂,
   - l'atome d'hydrogène ;
- R₂₅ est choisi parmi :
   - le radical 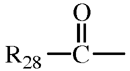
   - l'atome d'hydrogène ;
- R₂₄, R₂₆ et R₂₈, identiques ou différents, sont choisis parmi les radicaux hydrocarbonés en C₁₃-C₁₇, linéaires ou ramifiés, saturés ou insaturés, et de préférence parmi les radicaux alkyles et alcényles en C₁₃-C₁₇, linéaires ou ramifiés, saturés ou insaturés.

Avantageusement, les radicaux hydrocarbonés sont linéaires.

On peut citer par exemple les composés de formule (IX) tels que les sels (chlorure ou méthylsulfate notamment) de diacyloxyéthyl-diméthylammonium, de diacyloxyéthyl-hydroxyéthyl-méthylammonium, de monoacyloxyéthyl-dihydroxyéthyl-méthylammonium, de triacyloxyéthyl-méthylammonium, de monoacyloxyéthyl-hydroxyéthyl-diméthylammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle (méthyle ou éthyle de préférence), un sulfate de dialkyle (méthyle ou éthyle de préférence), le méthanesulfonate de méthyle, le para-toluènesulfonate de méthyle, la chlorhydrine du glycol ou du glycérol.

De tels composés sont par exemple commercialisés sous les dénominations DEHYQUART® par la société HENKEL, STEPANQUAT® par la société STEPAN, NOXAMIUM® par la société CECA, REWOQUAT® WE 18 par la société REWO-WITCO.

La composition selon l'invention peut contenir un mélange de sels de mono-, di- et triester d'ammonium quaternaire avec une majorité en poids de sels de diester.

Comme mélange de sels d'ammonium, on peut utiliser par exemple le mélange contenant 15 à 30 % en poids de méthylsulfate d'acyloxyéthyl-dihydroxyéthyl-méthylammonium, 45 à 60% de méthylsulfate de diacyloxyéthyl-hydroxyéthyl-méthylammonium et 15 à 30% de méthylsulfate de triacyloxyéthyl-méthylammonium, les radicaux acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4874554 et US-A-4137180.

Parmi les sels d'ammonium quaternaire de formule (VI), on préfère d'une part, les chlorures de tétraalkylammonium comme, par exemple, les chlorures de dialkyldiméthylammonium ou d'alkyltriméthylammonium dans lesquels le radical alkyle comporte environ de 12 à 22 atomes de carbone, en particulier les chlorures de béhényltriméthylammonium, de distéaryldiméthylammonium, de cétyltriméthylammonium, de benzyldiméthylstéarylammonium ou encore, d'autre part, le chlorure de palmitylamidopropyltriméthylammonium ou le chlorure de stéaramidopropyldiméthyl-(myristyl acétate)-ammonium commercialisé sous la dénomination CERAPHYL® 70 par la société VAN DYK.

La cire ou les cires éventuellement présentes dans la composition ayant un pH acide utilisée dans le procédé selon l'invention sont choisies notamment, parmi la cire de Carnauba, la cire de Candellila, et la cire d'Alfa, la cire de paraffine, l'ozokérite, les cires végétales comme la cire d'olivier, la cire de riz, la cire de jojoba hydrogénée ou les cires absolues de fleurs telles que la cire essentielle de fleur de cassis vendue par la société BERTIN (France), les cires animales comme les cires d'abeilles, ou les cires d'abeilles modifiées (cerabellina) ; d'autres cires ou matières premières cireuses utilisables selon l'invention sont notamment les cires marines telles que celle vendue par la Société SOPHIM sous la référence M82, les cires de polyéthylène ou de polyoléfines en général.

Les silicones pouvant être comprises dans la composition ayant un pH acide utilisée selon l'invention peuvent être choisies parmi toutes les silicones mentionnées précédemment au sujet de la composition alcaline.

Généralement, le pH de la composition ayant un pH acide utilisée dans le procédé selon l'invention est compris entre 2,0 et 4,5.

Le pH de la composition acide est ajusté au moyen d'un ou plusieurs d'agents acidifiants tels que par exemple l'acide chlorhydrique, l'acide acétique, l'acide lactique, l'acide borique, l'acide citrique et l'acide phosphorique.

Comme expliqué précédemment, le ou les agents réducteurs et le ou les céramides présents respectivement dans la composition alcaline et la composition ayant un pH acide sont placés dans un milieu cosmétiquement acceptable.

Ainsi, le véhicule des compositions alcaline et acide utilisées dans le procédé selon l'invention est de préférence un milieu aqueux constitué par de l'eau et peut avantageusement contenir des solvants organiques acceptables sur le plan cosmétique, dont plus particulièrement, des alcools tels que l'alcool éthylique, l'alcool isopropylique, l'alcool benzylique, et l'alcool phényléthylique, ou des polyols ou éthers de polyols tels que, par exemple, le glycérol, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol. Les solvants organiques peuvent alors être présents dans des concentrations comprises entre environ 0,1 et 20% et, de préférence, entre environ 1 et 10% en poids par rapport au poids total de la composition.

Comme expliqué précédemment, après l'application sur les cheveux de la composition ayant un PH acide, on rince les cheveux, généralement à l'eau.

Selon un mode de réalisation particulier de l'invention, après l'application de la composition ayant un pH acide et avant le rinçage, on laisse reposer les cheveux sous chauffage (casque chauffant) ou sous atmosphère de vapeur d'eau (casque à vapeur).

Selon un autre mode de réalisation particulier de l'invention, on applique sur les cheveux, après l'application de la composition ayant un pH acide et avant le rinçage, une composition comprenant une ou plusieurs huiles.

Comme huiles pouvant être appliquées sur les cheveux comme expliqué précédemment, on peut citer par exemple :
- les huiles hydrocarbonées d'origine animale, telles que le perhydrosqualène;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité ;

- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que Parléam® ; les isoparaffines comme l'isohexadécane et l'isodécane.
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les alcools gras alcoylés et notamment éthoxylés tels que l'oleth-12 ;
- les huiles fluorées partiellement hydrocarbonées et/ou siliconées comme celles décrites dans le document JP-A-2-295912 ; comme huiles fluorées, on peut citer aussi le perfluorométhylcyclopentane et le perfluoro-1,3 diméthylcyclohexane, vendus sous les dénominations de "FLUTEC PC1®" et "FLUTEC PC3®" par la Société BNFL Fluorochemicals ; le perfluoro-1,2-diméthylcyclobutane ; les perfluoroalcanes tels que le dodécafluoropentane et le tétradécafluorohexane, vendus sous les dénominations de "PF 5050®" et "PF 5060®" par la Société 3M, ou encore le bromoperfluorooctyle vendu sous la dénomination "FORALKYL®" par la Société Atochem ; le nonafluoro-méthoxybutane et le nonafluoroéthoxyisobutane ; les dérivés de perfluoromorpholine, tels que la 4-trifluorométhyl perfluoromorpholine vendue sous la dénomination "PF 5052®" par la Société 3M ;
- les huiles de silicone comme les polyméthylsiloxanes (PDMS) volatiles ou non à chaîne siliconée linéaire ou cyclique, liquides ou pâteux à température ambiante, notamment les cyclopoly-diméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthyl phényl siloxanes ;
- leurs mélanges.

Suite à l'étape d'application de la composition alcaline comprenant un ou plusieurs agents réducteurs et à l'étape de rinçage de cette composition, les cheveux subissent une oxydation. Cette oxydation peut être réalisée à l'aide d'une composition oxydante ou par action de l'oxygène de l'air. De préférence, les cheveux subissent une oxydation naturelle par l'oxygène de l'air.

Par conséquent, de façon préférée, le procédé selon l'invention ne comporte aucune étape d'application sur les cheveux d'un ou plusieurs agents oxydants puissants, tels que l'eau oxygénée ou les bromates par exemples. La composition ayant un pH acide comprenant le ou les céramides est exempte de tout agent oxydant.

Selon un mode de réalisation préféré, la composition alcaline est exempte de céramide.

Le procédé selon l'invention permet d'assouplir la fibre capillaire de façon durable. Aucune modification notable de l'esthétique de la coiffure, tel que le frisage ou le lissage, n'est apportée, contrairement au résultat obtenu lors d'un procédé classique de mise en forme permanente par mise sous tension mécanique des cheveux et utilisation d'une composition réductrice puis oxydante. De plus, le toucher de la chevelure reste naturel.

L'invention est illustrée par l'exemple qui suit.

### Exemple

On effectue une opération d'assouplissement durable d'une chevelure asiatique au moyen du procédé selon l'invention. On utilise pour cela une composition alcaline et une composition ayant un pH acide.

La formulation des compositions est la suivante.

**Composition alcaline (en % en poids) :**

| | |
|---|---|
| N-acétyl-cystéine | 1,2 |
| Arginine | 2 |
| Hydroxyéthyl cellulose | 1,2 |
| Silicone cationique (Quaternium-80) | 0,75 |
| Glycérol | 10 |
| Parfum | 0,2 |
| Eau | 84,65 |

Le pH de la composition alcaline est de 8,5.

**Composition ayant un pH acide (en % en poids) :**

| | |
|---|---|
| N-oléoyldihydrosphingosine | 0,1 |
| Chlorure de béhényl triméthyl ammonium à 80% en solution eau/ isopropanol (GENAMIN KDMP de CLARIANT) | 2 |
| Cire de candellila | 0,3 |
| DOW CORNING® 2-8299 CATIONIC EMULSION | 1,5 |
| Acide citrique | qs pH 3 |
| Eau | qs 100 |

On applique sur la chevelure la composition alcaline. Puis on rince.

On applique alors la composition ayant un pH acide pour traiter le cheveu en profondeur. Puis on rince.

On obtient une chevelure assouplie de façon durable.

## Revendications

1. Procédé de traitement capillaire comprenant les étapes consistant à :
- appliquer sur les cheveux une composition alcaline comprenant dans un milieu cosmétiquement acceptable un ou plusieurs agents réducteurs, le ou les agents réducteurs représentant entre 0,1 et 4% en poids du poids total de la composition alcaline,
- rincer les cheveux,
- appliquer sur les cheveux une composition ayant un pH acide contenant dans un milieu cosmétiquement acceptable un ou plusieurs céramides, ladite composition étant exempte de tout agent oxydant, puis
- rincer les cheveux.

2. Procédé selon la revendication 1 **caractérisé en ce que** le pH de la composition alcaline est compris entre 7,5 et 9,5.

3. Procédé selon la revendication 1 ou 2 **caractérisé en ce que** le ou les composés réducteurs représentent entre 1 et 2%, en poids du poids total de la composition alcaline.

4. Procédé selon l'une quelconque des revendications précédentes **caractérisé en ce que** le ou les agents réducteurs sont choisis parmi les agents réducteurs de formule
H(X')_{q}(R⁰)ᵣ
dans laquelle X' représente P, S ou SO₂, q vaut 0 ou 1, r vaut 1 ou 2 et R⁰ est un radical (C₁-C₂₀)hydrocarboné, linéaire, ramifié, saturé, insaturé, éventuellement interrompu par un hétéroatome, et comportant éventuellement des substituants choisis parmi un groupement hydroxy, un groupement halogéné, un groupement amine ou un groupement carboxy, un groupement ((C₁-C₃₀) alcoxy)carbonyle, un groupement amido, un groupement ((C₁-C₃₀)alkyl)amino carbonyle, un groupement (C₁₋C₃₀)acyle) amino, un groupement mono ou dialkylamino, un groupement mono ou dihydroxylamino, ou l'un de ses sels en combinaison avec une base.

5. Procédé selon la revendication 4 **caractérisé en ce que** le ou les agents réducteurs sont choisis parmi l'acide thioglycolique, l'acide thiolactique, le monothioglycolate de glycérol, la cystéamine, la N-acétyl-cystéamine, la N-propionyl-cystéamine, la cystéine, la N-acétyl-cystéine, l'acide thiomalique, la panthétéine, l'acide 2,3-dimercaptosuccinique, les N-(mercaptoalkyl)-ω-hydroxyalkylamides, les N-mono ou N,N-dialkylmercapto-4-butyramides, les aminomercapto-alkylamides, les dérivés des acides N-(mercaptoalkyl)succinamiques et des N-(mercaptoalkyl)succinimides, les alkylamino mercaptoalkylamides, le mélange azéotrope de thioglyconate de 2-hydroxypropyle et de thioglycolate de (2-hydroxy-1-méthyl)éthyle, les mercaptoalkylaminoamides, les N-mercapto-alkylalcanediamides et les dérivés d'acide formamidine sulfinique, et leurs sels.

6. Procédé selon la revendication 5 **caractérisé en ce que** le ou les agents réducteurs sont choisis parmi la cystéine ou la N-acétyl-cystéine.

7. Procédé selon l'une quelconque des revendications précédentes **caractérisé en ce que** le pH de la composition ayant un pH acide est compris entre 2,0 et 4,5.

8. Procédé selon l'une quelconque des revendications précédentes **caractérisé en ce que** le ou les céramides sont choisis parmi les composés de formule (I) suivante : 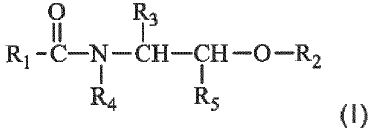 dans laquelle :
- R₁ désigne soit un radical hydrocarboné, linéaire ou ramifié, saturé ou insaturé en C₉-C₃₀, ce radical pouvant être substitué par un ou plusieurs groupements hydroxyle, ces groupements hydroxyle étant éventuellement estérifiés par un acide gras saturé ou insaturé en C₁₆-C₃₀ ; soit un radical R"-(NR-CO)-R', où R désigne un atome d'hydrogène ou un radical hydrocarboné en C₁-C₁₀ mono ou polyhydroxylé, préférentiellement monohydroxylé, R' et R" sont des radicaux hydrocarbonés dont la somme des atomes de carbone est comprise entre 9 et 30, R' étant un radical divalent :
- R₂ désigne un atome d'hydrogène ou un radical (glycosyle)ₙ, (galactosyle)ₘ ou sulfogalactosyle, dans lesquels n est un nombre entier variant de 1 à 4 inclusivement et m est un nombre entier variant de 1 à 8 inclusivement ;
- R₃ désigne un atome d'hydrogène ou un radical hydrocarboné en C₁₆-C₂₇, saturé ou insaturé, ce radical pouvant être substitué par un ou plusieurs radicaux alkyle en C₁-C₁₄ ; R₃ peut également désigner un radical α-hydroxyalkyle en C₁₅-C₂₆ dont le groupement hydroxyle peut éventuellement être estérifié par un α-hydroxyacide en C₁₆-C₃₀ ;
- R₄ désigne un atome d'hydrogène, un radical hydrocarboné en C₁₆-C₂₇, saturé ou insaturé, ou un radical -CH₂-CHOH-CH₂-O-R₆ dans lequel R₆ désigne un radical hydrocarboné en C₁₀-C₂₆ ;
- R₅ désigne un atome d'hydrogène ou un radical hydrocarboné en C₁-C₄ mono ou polyhydroxylé.

9. Procédé selon la revendication 8, **caractérisé en ce que** le ou les céramides sont choisis parmi les composés de formule (I) dans laquelle R₁ désigne un radical alkyle saturé ou insaturé dérivé d'acides gras en C₁₆-C₂₂ éventuellement hydroxylé ; R₂ désigne un atome d'hydrogène ; et R₃ désigne un radical linéaire saturé en C₁₅ éventuellement hydroxylé.

10. Procédé selon la revendication 9, **caractérisée en ce que** le ou les céramides sont choisis parmi :
- la N-linoléoyldihydrosphingosine,
- la N-oléoyldihydrosphingosine,
- la N-palmitoyldihydrosphingosine,
- la N-stéaroyldihydrosphingosine,
- la N-béhénoyldihydrosphingosine,
- la N-2-hydroxypalmitoyldihydrosphingosine,
- la N-stéaroylphytosphingosine,
ou les mélanges de ces composés.

11. Procédé selon la revendication 10, **caractérisée en ce que** le céramide est la N-oléoyldihydrosphingosine.

12. Procédé selon l'une quelconque des revendications précédentes **caractérisé en ce que** le ou les céramides représentent de 0,001 à 20%, de préférence de 0,01 à 10%, et mieux de 0,05 à 1% en poids du poids total de la composition ayant un pH acide.

13. Procédé selon l'une quelconque des revendications précédentes **caractérisé en ce que**, après l'application de la composition ayant un pH acide et avant le rinçage, on laisse reposer les cheveux sous chauffage ou sous atmosphère de vapeur d'eau.

14. Procédé selon l'une quelconque des revendications précédentes **caractérisé en ce que**, après l'application de la composition ayant un pH acide et avant le rinçage, on applique une composition comprenant une ou plusieurs huiles.

## Patentansprüche

1. Verfahren zur Haarbehandlung, bei dem man:
- auf die Haare eine alkalische Zusammensetzung, die in einem kosmetisch unbedenklichen Medium ein oder mehrere Reduktionsmittel umfasst, aufbringt, wobei das bzw. die Reduktionsmittel zwischen 0,1 und 4 Gew.-% des Gesamtgewichts der alkalischen Zusammensetzung ausmachen,
- die Haare spült,
- auf die Haare eine Zusammensetzung mit einem sauren pH-Wert, die in einem kosmetisch unbedenklichen Medium ein oder mehrere Ceramide enthält, aufbringt, wobei die Zusammensetzung frei von jeglichem Oxidationsmittel ist, und dann
- die Haare spült.

2. Verfahren nach Anspruch 1, **dadurch gekennzeichnet, dass** der pH-Wert der alkalischen Zusammensetzung zwischen 7,5 und 9,5 liegt.

3. Verfahren nach Anspruch 1 oder 2, **dadurch gekennzeichnet, dass** die reduzierende Verbindung bzw. die reduzierenden Verbindungen zwischen 1 und 2 Gew.-% des Gesamtgewichts der alkalischen Zusammensetzung ausmachen.

4. Verfahren nach einem der vorhergehenden Ansprüche, **dadurch gekennzeichnet, dass** das bzw. die Reduktionsmittel aus Reduktionsmitteln der Formel
H(X')_{q}(R⁰)ᵣ
wobei X' für P, S oder SO₂ steht, q gleich 0 oder 1 ist, r gleich 1 oder 2 ist und R⁰ für einen linearen oder verzweigten, gesättigten oder ungesättigten (C₁-C₂₀)Kohlenwasserstoffrest, der gegebenenfalls durch ein Heteroatom unterbrochen ist und gegebenenfalls Substituenten, die aus einer Hydroxygruppe, einer halogenierten Gruppe, einer Amingruppe oder einer Carboxygruppe, einer ((C₁-C₃₀)Alkoxy)carbonylgruppe, einer Amidogruppe, einer ((C₁-C₃₀)Alkyl)aminocarbonylgruppe, einer ((C₁-C₃₀)Acyl)aminogruppe, einer Monoalkylamino- oder Dialkylaminogruppe, einer Monohydroxylamino- oder Dihydroxylaminogruppe ausgewählt sind, umfasst, steht, oder einem Salz davon in Kombination mit einer Base ausgewählt werden.

5. Verfahren nach Anspruch 4, **dadurch gekennzeichnet, dass** das bzw. die Reduktionsmittel aus Thioglykolsäure, Thiomilchsäure, Glycerinmonothioglycolat, Cysteamin, N-Acetylcysteamin, N-Propionylcysteamin, Cystein, N-Acetylcystein, Thioäpfelsäure, Panthetein, 2,3-Dimercaptobernsteinsäure, N-(Mercaptoalkyl)-ω-hydroxyalkylamiden, N-Mono- oder N,N-Dialkylmercapto-4-butyramiden, Aminomercaptoalkylamiden, Derivaten von N-(Mercaptoalkyl)succinamidsäuren und N-(Mercaptoalkyl)-succinimiden, Alkylaminomercaptoalkylamiden, dem azeotropen Gemisch von 2-Hydroxypropylthioglykonat und (2-Hydroxy-1-methyl)ethylthioglykolat, Mercaptoalkylaminoamiden, N-Mercaptoalkylalkandiamiden und Formamidinsulfinsäurederivaten und Salzen davon ausgewählt werden.

6. Verfahren nach Anspruch 5, **dadurch gekennzeichnet, dass** das bzw. die Reduktionsmittel aus Cystein oder N-Acetylcystein ausgewählt werden.

7. Verfahren nach einem der vorhergehenden Ansprüche, **dadurch gekennzeichnet, dass** der pH-Wert der Zusammensetzung mit einem sauren pH-Wert zwischen 2,0 und 4,5 liegt.

8. Verfahren nach einem der vorhergehenden Ansprüche, **dadurch gekennzeichnet, dass** das bzw. die Ceramide aus den Verbindungen der folgenden Formel (I) ausgewählt werden: 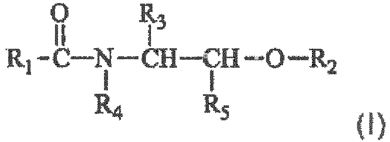 in der:
- R₁ für einen linearen oder verzweigten, gesättigten oder ungesättigten C₉-C₃₀-Kohlenwasserstoffrest, wobei dieser Rest durch eine oder mehrere Hydroxylgruppen substituiert sein kann, wobei diese Hydroxylgruppen gegebenenfalls durch eine gesättigte oder ungesättigte C₁₆-C₃₀-Fettsäure verestert sein können; oder einen Rest R"-(NR-CO)-R', wobei R für ein Wasserstoffatom oder einen mono- oder polyhydroxylierten, vorzugsweise monohydroxylierten, C₁-C₁₀-Kohlenwasserstoffrest steht und R' und R" für Kohlenwasserstoffreste mit einer Summe von Kohlenstoffatomen zwischen 9 und 30 stehen, wobei R' für einen zweiwertigen Rest steht, steht;
- R₂ für ein Wasserstoffatom oder einen (Glycosyl)ₙ-, (Galactosyl)m- oder Sulfogalactosylrest steht, wobei n für eine ganze Zahl von 1 bis 4 inklusive steht und m für eine ganze Zahl von 1 bis 8 inklusive steht;
- R₃ für ein Wasserstoffatom oder einen gesättigten oder ungesättigten C₁₆-C₂₇-Kohlenwasserstoffrest steht, wobei dieser Rest durch einen oder mehrere C₁-C₁₄-Alkylreste substituiert sein kann; R₃ auch für einen C₁₅-C₂₆-α-Hydroxyalkylrest, dessen Hydroxylgruppe gegebenenfalls durch eine C₁₆-C₃₀-α-Hydroxysäure verestert sein kann, stehen kann;
- R₄ für ein Wasserstoffatom, einen gesättigten oder ungesättigten C₁₆-C₂₇-Kohlenwasserstoffrest oder einen -CH₂-CHOH-CH₂-O-R₆-Rest steht, wobei R₆ für einen C₁₀-C₂₆-Kohlenwasserstoffrest steht;
- R₅ für ein Wasserstoffatom oder einen mono- oder polyhydroxylierten C₁-C₄-Kohlenwasserstoffrest steht.

9. Verfahren nach Anspruch 8, **dadurch gekennzeichnet, dass** das bzw. die Ceramide aus den Verbindungen der Formel (I), in der R₁ für einen gesättigten oder ungesättigten Alkylrest, der sich von C₁₆-C₂₂-Fettsäuren ableitet und gegebenenfalls hydroxyliert ist, steht; R₂ für ein Wasserstoffatom steht und R₃ für einen gegebenenfalls hydroxylierten C₁₅-Kohlenwasserstoffrest steht; ausgewählt werden.

10. Verfahren nach Anspruch 9, **dadurch gekennzeichnet, dass** das bzw. die Ceramide aus:
N-Linoleoyldihydrosphingosin,
N-Oleoyldihydrosphingosin,
N-Palmitoyldihydrosphingosin,
N-Stearoyldihydrosphingosin,
N-Behenoyldihydrosphingosin,
N-2-Hydroxypalmitoyldihydrosphingosin,
N-Stearoylphytosphingosin
oder Mischungen dieser Verbindungen ausgewählt werden.

11. Verfahren nach Anspruch 10, **dadurch gekennzeichnet, dass** es sich bei dem Ceramid um N-Oleoyldihydrosphingosin handelt.

12. Verfahren nach einem der vorhergehenden Ansprüche, **dadurch gekennzeichnet, dass** das bzw. die Ceramide 0,001 bis 20 Gew.-%, vorzugsweise 0,01 bis 10 Gew.-% und noch besser 0,05 bis 1 Gew.-% des Gesamtgewichts der Zusammensetzung mit einem sauren pH-Wert ausmachen.

13. Verfahren nach einem der vorhergehenden Ansprüche, **dadurch gekennzeichnet, dass** man nach dem Aufbringen der Zusammensetzung mit einem sauren pH-Wert und vor dem Spülen die Haare unter Erhitzen oder unter Wasserdampfatmosphäre ruhen lässt.

14. Verfahren nach einem der vorhergehenden Ansprüche, **dadurch gekennzeichnet, dass** man nach dem Aufbringen der Zusammensetzung mit einem sauren pH-Wert und vor dem Spülen eine Zusammensetzung, die ein oder mehrere Öle umfasst, aufbringt.

## Claims

1. Hair treatment method comprising the stages consisting in:
- applying, to the hair, an alkaline composition comprising, in a cosmetically acceptable medium, one or more reducing agents, the reducing agent or agents representing from 0.1 to 4% by weight of the total weight of the alkaline composition,
- rinsing the hair,
- applying, to the hair, a composition having an acid pH containing, in a cosmetically acceptable medium, one or more ceramides, the said composition being devoid of any oxidizing agent, then
- rinsing the hair.

2. Method according to Claim 1, **characterized in that** the pH of the alkaline composition is between 7.5 and 9.5.

3. Method according to Claim 1 or 2, **characterized in that** the reducing compound or compounds represent between 1 and 2% by weight of the total weight of the alkaline composition.

4. Method according to any one of the preceding claims, **characterized in that** the reducing agent or agents are chosen from the reducing agents of formula
H(X')_{q}(R⁰)ᵣ
in which X' represents P, S or SO₂, q has a value 0 or 1, r has a value 1 or 2 and R⁰ is a saturated or unsaturated and linear or branched C₁-C₂₀ hydrocarbon radical optionally interrupted by a heteroatom and optionally comprising substituents chosen from a hydroxyl group, a halogenated group, an amine group or a carboxyl group, a (C₁-C₃₀)alkoxycarbonyl group, an amido group, a (C₁-C₃₀) alkylaminocarbonyl group, a (C₁-C₃₀)acylamino group, a mono- or dialkylamino group or a mono- or dihydroxyamino group, or a salt thereof in combination with a base.

5. Method according to Claim 4, **characterized in that** the reducing agent or agents are chosen from thioglycolic acid, thiolactic acid, glycerol monothiolglycolate, cysteamine, N-acetylcysteamine, N-propionylcysteamine, cysteine, N-acetylcysteine, thiomalic acid, pantetheine, 2,3-dimercaptosuccinic acid, N-(mercaptoalkyl)-ω-hydroxyalkylamides, N-monoalkyl- or N,N-dialkylmercapto-4-butyramides, aminomercapto-alkylamides, derivatives of N-(mercaptoalkyl)succinamic acids and of N-(mercaptoalkyl)succinimides, alkylaminomercaptoalkylamides, the azeotropic mixture of 2-hydroxypropyl thioglycolate and 2-hydroxy-1-methylethyl thioglycolate, mercaptoalkylaminoamides, N-mercaptoalkylalkanediamides and formamidinesulfinic acid derivatives, and their salts.

6. Method according to Claim 5, **characterized in that** the reducing agent or agents are chosen from cysteine or N-acetylcysteine.

7. Method according to any one of the preceding claims, **characterized in that** the pH of the composition having an acid pH is between 2.0 and 4.5.

8. Method according to any one of the preceding claims, **characterized in that** the ceramide or ceramides are chosen from the compounds of following formula (I): 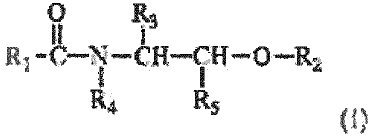 in which:
- R₁ denotes either a saturated or unsaturated and linear or branched C₉-C₃₀ hydrocarbon radical, it being possible for this radical to be substituted by one or more hydroxyl groups, these hydroxyl groups optionally being esterified by a saturated or unsaturated C₁₆-C₃₀ fatty acid; or a R"-(NR-CO)-R' radical, where R denotes a hydrogen atom or a mono- or polyhydroxylated, preferably monohydroxylated, C₁-C₁₀ hydrocarbon radical and R' and R" are hydrocarbon radicals, the sum of the carbon atoms of which is between 9 and 30, R' being a divalent radical;
- R₂ denotes a hydrogen atom or a (glycosyl)ₙ, (galactosyl)ₘ or sulfogalactosyl radical, in which n is an integer varying from 1 to 4 inclusive and m is an integer varying from 1 to 8 inclusive;
- R₃ denotes a hydrogen atom or a saturated or unsaturated C₁₆-C₂₇ hydrocarbon radical, it being possible for this radical to be substituted by one or more C₁-C₁₄ alkyl radicals; R₃ can also denote a C₁₅-C₂₆ α-hydroxyalkyl radical, the hydroxyl group of which can optionally be esterified by a C₁₆-C₃₀ α-hydroxy acid;
- R₄ denotes a hydrogen atom, a saturated or unsaturated C₁₆-C₂₇ hydrocarbon radical or a -CH₂-CHOH-CH₂-O-R₆ radical in which R₆ denotes a C₁₀-C₂₆ hydrocarbon radical;
- R₅ denotes a hydrogen atom or a mono- or polyhydroxylated C₁-C₄ hydrocarbon radical.

9. Method according to Claim 8, **characterized in that** the ceramide or ceramides are chosen from the compounds of formula (I) in which R₁ denotes a saturated or unsaturated and optionally hydroxylated alkyl radical derived from C₁₆-C₂₂ fatty acids; R₂ denotes a hydrogen atom; and R₃ denotes a saturated, linear and optionally hydroxylated C₁₅ radical.

10. Method according to Claim 9, **characterized in that** the ceramide or ceramides are chosen from:
- N-linoleoyldihydrosphingosine,
- N-oleoyldihydrosphingosine,
- N-palmitoyldihydrosphingosine,
- N-stearoyldihydrosphingosine,
- N-behenoyldihydrosphingosine,
- N-(2-hydroxypalmitoyl)dihydrosphingosine,
- N-stearoylphytosphingosine,
or the mixtures of these compounds.

11. Method according to Claim 10, **characterized in that** the ceramide is N-oleoyldihydrosphingosine.

12. Method according to any one of the preceding claims, **characterized in that** the ceramide or ceramides represent from 0.001 to 20%, preferably from 0.01 to 10% and better still from 0.05 to 1% by weight of the total weight of the composition having an acid pH.

13. Method according to any one of the preceding claims, **characterized in that**, after the application of the composition having an acid pH and before the rinsing, the hair is left to stand under heating or under an atmosphere of steam.

14. Method according to any one of the preceding claims, **characterized in that**, after the application of the composition having an acid pH and before the rinsing, a composition comprising one or more oils is applied.
